# FASCICULE DE BREVET EUROPEEN

(11) **EP 1 250 101 B1**
(45) Date de publication et mention de la délivrance du brevet: **21.12.2005**
(21) Numéro de dépôt: 01903979.1
(22) Date de dépôt: 26.01.2001
(51) Int. Cl.: A61B 17/70

(54) **DISPOSITIF DE LIAISON INTERVERTEBRALE AVEC UNE BARRE DE CONNEXION POUR LA FIXATION D'UNE TIGE DE LIAISON**
ZWISCHENWIRBELVERBINDUNGSEINRICHTUNG MIT EINER STANGE ZUR FIXIERUNG DES VERBINDUNGSSTABES
INTERVERTEBRAL LINKING DEVICE WITH CONNECTING BAR FOR FIXING A LINKING ROD

(30) Priorité: 27.01.2000 FR 0001071
(43) Date de publication de la demande: 23.10.2002
(73) Titulaire: SCIENT'X, 78284 Guyancourt (FR); Bone & Joint Research S.A., 3644 Kayl (LU)
(72) Inventeur: MUNTING, Everard, B-1390 Biez (BE)
(74) Mandataire: Thibault, Jean-Marc
(86) Numéro de dépôt international: PCT/FR2001/000259
(87) Numéro de publication internationale: WO 2001/054597

(56) Documents cités:
- FR-A- 2 781 359
- US-A- 5 437 669
- US-A- 5 437 671
- US-A- 5 613 968

## Description

La présente invention concerne le domaine technique de l'ostéosynthèse au sens général et elle vise les systèmes ou les dispositifs assurant une liaison intervertébrale adaptée pour stabiliser le rachis ou pour corriger les déformations du rachis, en particulier, les déformations scoliotiques.

Dans l'état de la technique, il est connu de nombreux dispositifs de liaison intervertébrale. D'une manière classique, un tel dispositif comporte des éléments d'ancrage osseux, tels que par exemple des vis d'implantation pédiculaire ou des crochets vertébraux simples ou doubles équipés chacun d'une tête de fixation pour une tige de liaison reliant lesdits implants entre eux. Les éléments d'ancrage osseux sont répartis le long de la zone du rachis à traiter pour permettre le montage de deux tiges de liaison s'étendant sensiblement parallèlement l'une à l'autre en étant disposées de chaque côté des apophyses épineuses des vertèbres. Parfois, un entretoisement transversal entre les tiges de liaison est mis en oeuvre pour rendre plus stable la construction ainsi réalisée.

Une des difficultés pour la mise en place d'un tel dispositif de liaison intervertébrale concerne la connexion entre les tiges de liaison intervertébrale et les éléments d'ancrage osseux. Une telle connexion est rendue délicate en raison, notamment, du non alignement dans le plan frontal des éléments d'ancrage osseux, de la différence de hauteur entre les éléments d'ancrage osseux et de l'angulation imposée par l'anatomie du rachis à traiter. De telles difficultés de connexion de ces tiges de liaison font apparaître fréquemment des contraintes mécaniques qui sont qui viennent s'ajouter à celles engendrées par l'action correctrice ou stabilisatrice appliquée, et qui sont susceptibles d'entraîner l'arrachement au moins partiel des éléments d'ancrage osseux, voire une cassure d'une des parties constitutives d'un tel dispositif de liaison intervertébrale.

Pour tenter de surmonter ces inconvénients, le brevet US 5 437 671 décrit un dispositif de liaison intervertébrale comportant une barre de connexion transversale, fixée sur des éléments d'ancrage osseux, équipée de deux systèmes de fixation pour des tiges de liaison. Chaque système de fixation d'une tige de liaison est monté sur la barre de connexion par l'intermédiaire de deux crochets.

Il doit être noté qu'un tel système de fixation ne comporte pas de moyens permettant d'assurer son blocage dans une position fixe déterminée le long de cette barre de connexion. De plus, ce document ne décrit pas le montage de la barre de connexion avec une possibilité de rotation autour de son axe et de blocage dans une position fixe déterminée par rapport aux vis d'ancrage.

Un tel dispositif de liaison intervertébrale ne permet donc pas une adaptation de la tige de liaison sur la barre de connexion selon les trois plans de l'espace. De plus, un tel dispositif ne permet pas d'obtenir une structure rigide entre les tiges de liaison et les barres transversales de connexion.

L'analyse des solutions antérieures connues conduit à constater qu'il apparaît le besoin de disposer d'un dispositif de liaison intervertébrale conçu pour faciliter, eu égard au décalage angulaire imposé par le rachis, la connexion entre les éléments d'ancrage osseux et une tige de liaison intervertébrale, tout en limitant les contraintes mécaniques imposées aux diverses parties constitutives d'un tel dispositif de liaison, notamment en supprimant les contraintes générées par la simple liaison des différents éléments d'ancrage dont l'alignement selon les trois plans de l'espace peut être imparfait.

L'objet de l'invention vise donc à satisfaire ce besoin en proposant un dispositif de liaison intervertébrale selon la revendication 1.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.

La **fig. 1** est une vue en perspective montrant un exemple d'application pour un dispositif de liaison intervertébrale conforme à l'invention.

La **fig. 2** est une vue en perspective montrant un exemple préféré de réalisation d'un ensemble de fixation.

La **fig. 3** est une vue en perspective d'un système de fixation faisant partie d'un ensemble de fixation illustré à la **fig. 2.**

La **fig. 4** est une vue en coupe élévation du système de fixation illustré à la fig. 3.

Les **fig. 5** et **6** sont des vues, respectivement en perspective et de côté, d'un autre exemple de réalisation d'un système de fixation.

Les **fig. 7** et **8** sont des vues, respectivement en perspective et en coupe-élévation d'un autre exemple de réalisation d'un système de fixation.

La **fig. 9** est une vue en perspective montrant une variante de réalisation du dispositif de liaison conforme à l'invention.

La **fig. 10** est une vue en perspective, partiellement arrachée, montrant un détail caractéristique de la variante illustrée à la **fig. 9.**

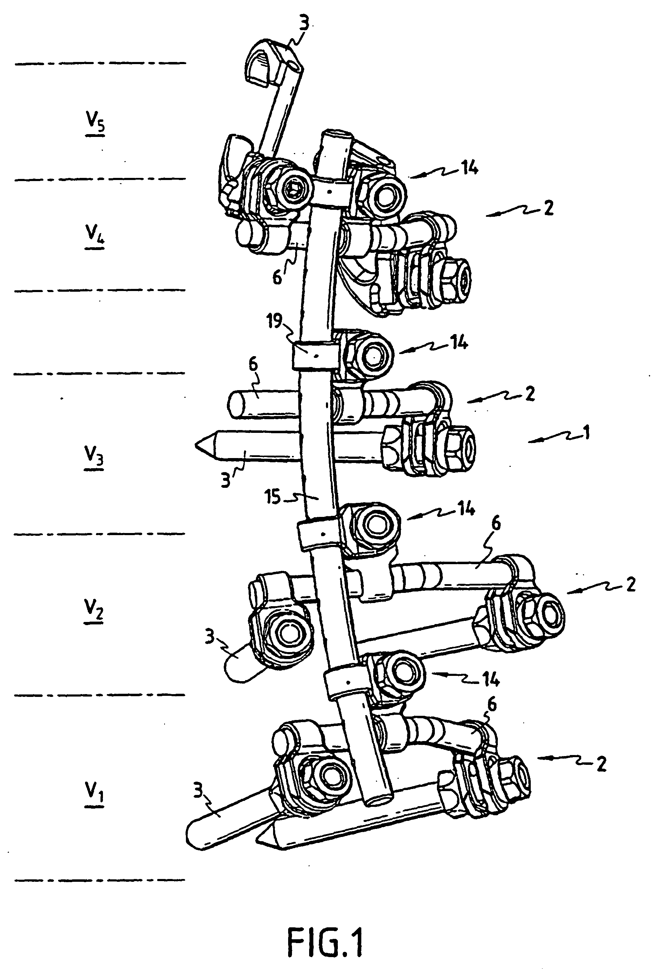

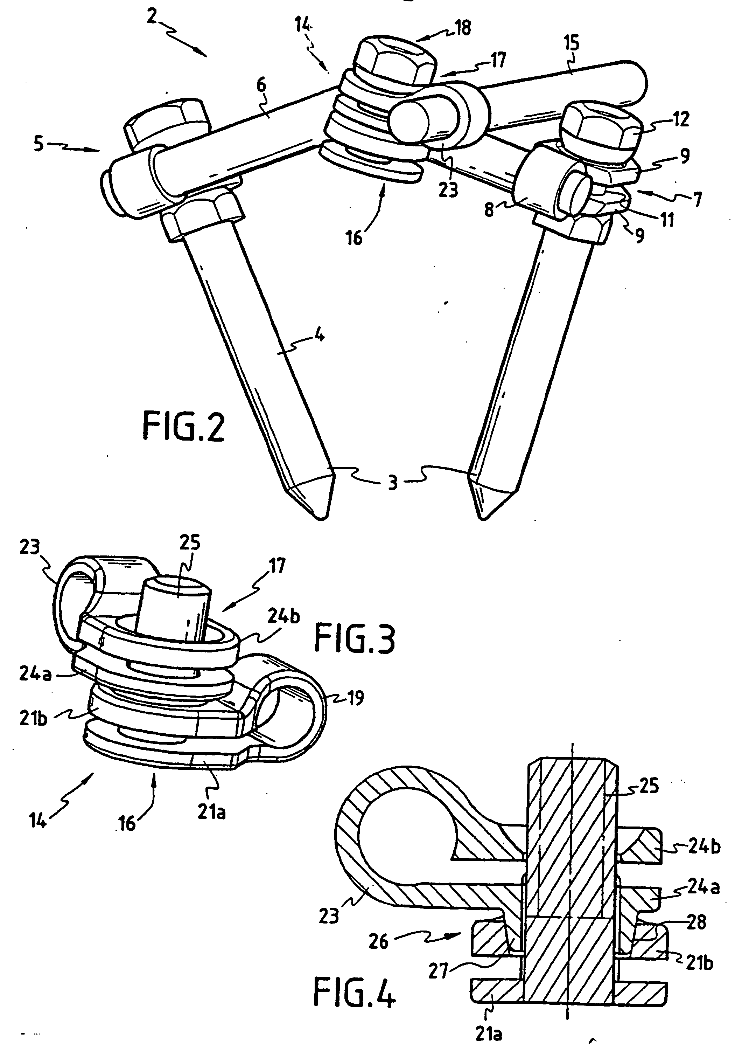

Tel que cela ressort plus précisément de la **fig. 1,** l'objet de l'invention concerne un dispositif **1** de liaison intervertébrale comportant au moins un, et dans l'exemple illustré, quatre ensembles de fixation **2** adaptés pour être montés sur des vertèbres représentées schématiquement, à titre illustratif, par des segments **V**_{**1**} à **V**_{**5**}**.** Chaque ensemble de fixation **2** comporte au moins un et, de préférence, deux éléments d'ancrage osseux **3,** destinés à être montés sur deux vertèbres adjacentes ou, avantageusement, sur une même vertèbre. Dans l'exemple illustré à la **fig. 2,** chaque élément d'ancrage osseux **3** est constitué par une vis comportant une tige d'ancrage filetée **4** surmontée par une tête de fixation **5.**

Chaque ensemble de fixation **2** comporte une barre de fixation **6** montée sur l'élément ou les éléments d'ancrage **3** appartenant à chaque ensemble de fixation **2.** Selon la variante préférée de réalisation dans laquelle un ensemble de fixation **2** comporte une paire d'éléments d'ancrage **3,** les éléments d'ancrage **3** appartenant à une paire, sont reliés entre eux par l'intermédiaire d'une barre de connexion **6** formant ainsi un pont entre les éléments d'ancrage osseux **3.** Cette barre de connexion **6** est montée de toute manière appropriée sur les éléments d'ancrage osseux **3.** Dans un exemple préféré de réalisation, la barre de connexion **6** présente une section droite transversale circulaire et se trouve montée sur chaque élément d'ancrage osseux **3** par l'intermédiaire de moyens **7** assurant un guidage en rotation de la barre **6** autour de son axe et un blocage dans une position fixe. De préférence, la barre de connexion **6** présente au moins une courbure ou un cintrage dont la fonction apparaîtra plus précisément dans la suite de la description.

Dans l'exemple de réalisation illustré, les moyens de guidage et de blocage **7** sont constitués par un collier ouvert **8** définissant un logement de passage pour une partie terminale de la barre de connexion **6.** Chaque collier ouvert **8** est prolongé par deux branches **9** conformées chacune sous la forme d'une bague destinée à être insérée sur un prolongement fileté **11** faisant partie de la tête de fixation **5** de l'élément d'ancrage osseux **3.** L'extrémité du prolongement fileté **11** est destinée à recevoir un écrou **12** dont le vissage permet la fermeture du collier, conduisant au blocage de la barre de connexion **6** par rapport aux éléments d'ancrage osseux **3.**

Selon une autre caractéristique de l'invention, le dispositif de liaison intervertébrale **1** comporte un système **14** de fixation pour au moins une tige **15** de liaison intervertébrale. Le système de fixation **14** est monté sur la barre de connexion n6, de manière à permettre la fixation de la tige de liaison **15** dans une position déterminée par rapport aux éléments d'ancrage osseux **3.** Selon une forme avantageuse de réalisation, le système de fixation **14** comporte des moyens **16** adaptés pour assurer son montage dans une position déterminée suivant la longueur de la barre de connexion **6.** Ces moyens de montage **16** sont équipés de moyens **17** assurant la fixation de la tige de liaison **15.**

Dans l'exemple de réalisation illustré plus particulièrement aux **fig. 3** et **4,** les moyens de montage **16** du système de fixation **14** sont constitués par des moyens assurant au moins un coulissement d'un tel système de fixation, associés à des moyens **18** de blocage dudit système de fixation **14** dans une position fixe le long de la barre de connexion **6.** De préférence, les moyens de montage **16** sont conçus pour assurer également un pivotement du système de fixation **14** autour de l'axe de la barre de connexion **6.** Dans l'exemple de réalisation illustré aux fig. **3** et **4,** les moyens de montage **16** sont constitués par l'intermédiaire d'un collier ouvert **19,** dit premier, de passage de la barre de connexion **6.** Ce premier collier ouvert **19** se prolonge, de part et d'autre, par des branches **21a, 21b** dites respectivement, inférieure et supérieure sur lesquelles agissent, en tant que moyens de blocage **18,** des moyens de serrage, tels qu'un écrou dans l'exemple illustré, permettant d'assurer l'immobilisation du premier collier sur la barre de connexion **6.**

Les moyens de fixation **17** pour la tige de liaison **15** sont constitués également, dans un exemple préféré de réalisation, par l'intermédiaire d'un deuxième collier **23** de passage de la tige de liaison **15.** Ce deuxième collier ouvert **23** se prolonge, de part et d'autre, par des branches **24a** et **24b** respectivement dites inférieure et supérieure, sur lesquelles agissent des moyens de blocage permettant d'immobiliser ou de solidariser la tige de liaison **15** par rapport au système de fixation **14.** Selon une caractéristique préférée de réalisation, les moyens de blocage du deuxième collier **23** sont les mêmes que les moyens de blocage **18** du premier collier **19.** Ces moyens de blocage **18** sont constitués par une seule pièce, à savoir un écrou. A cet effet, le premier collier **19** comporte une branche inférieure **21a** munie d'une tige filetée **25** traversant la branche supérieure **21b,** conformée sous la forme d'une bague. Le deuxième collier **23** possède également des branches inférieure 24a et supérieure **24b** conformées sous la forme de bagues pour permettre leur emmanchement sur la tige filetée **25** qui est coiffée par l'écrou de serrage **18.**

Il doit être compris que les moyens de fixation **17** pour la tige de liaison **15,** sont montés sur les moyens de montage **16** avec une possibilité de rotation autour d'un axe de pivotement constitué par la tige filetée **25** s'étendant par exemple sensiblement perpendiculairement à la barre de connexion **6.** De préférence, les moyens de montage **16** et les moyens de fixation **17** sont aménagés pour comporter des moyens **26** assurant un calage angulaire entre eux. Dans l'exemple de réalisation illustré, les moyens **26** sont du type à emboîtement par cône, formés par un cône **27** s'étendant à partir de la face inférieure de la branche **24a** et coopérant avec un alésage **28** tronconique délimitant l'intérieur de la bague supérieure **21b.** Le serrage de l'écrou **18** conduit à l'enfoncement mutuel entre le cône **27** et l'alésage tronconique **28** assurant un blocage angulaire entre les deux colliers **19, 23.** Bien entendu, il peut être prévu de réaliser de manière différente les moyens de calage angulaire **26,** tels que par exemple par l'intermédiaire d'aspérités réalisées sur la face inférieure de la bague **24a** et sur la face supérieure de la bague **21b.**

Les avantages du dispositif de liaison intervertébrale **1** selon l'invention découlent directement de la description qui précède. Après l'implantation des dispositifs d'ancrage osseux **3** réalisés de toute manière appropriée, le ou les deux éléments d'ancrage osseux **3** de chaque ensemble de fixation **2** sont munis d'une barre de connexion **6** équipée de son système **14** de fixation pour une tige de liaison **15.** Dans le cas préféré où l'ensemble de fixation **2** comporte une paire d'éléments d'ancrage **3,** une barre de connexion **6,** équipée de son système de fixation **14,** est ainsi montée entre les éléments d'ancrage osseux **3** de la paire. Les éléments d'ancrage **3** de chaque paire sont installés sur deux vertèbres adjacentes (par exemple **V**_{**4**} et **V**_{**5**}**)** ou, de préférence, sur une même vertèbre (par exemple **V**_{**1**} et **V**_{**2**}).

Il est à noter que la barre de connexion **6** est adaptée pour rattraper les différences de hauteur existant entre les éléments d'ancrage osseux **3** des ensembles de fixation **2** adjacents. Un tel rattrapage peut être obtenu par la conformation donnée à la barre de connexion **6** et/ou par sa rotation sur elle-même permettant de décaler dans le plan sagittal, la hauteur pour la fixation de la tige de liaison **15.** La barre de connexion **6** est également conformée pour s'adapter à l'anatomie vertébrale et peut ainsi présenter, comme dans l'exemple illustré, une courbure concave dirigée vers la vertèbre. Une telle possibilité de réglage facilite les opérations de montage de la tige de liaison **15** entre deux ensembles de fixation adjacents, dans la mesure où deux points dans l'espace peuvent toujours être reliés par une même droite.

Par ailleurs, chaque dispositif de fixation **14** est monté de manière fixe sur la barre de connexion **6** dans une position choisie par rapport aux éléments d'ancrage osseux **3**. Il est à noter que le dispositif de fixation **14** peut être monté sur la barre de connexion **6** entre les éléments d'ancrage **3** ou en dehors de ces derniers, dans le cas où la barre de connexion **6** se prolonge sur un côté, autorisant ainsi une fixation latérale de la tige de liaison **15.** Une telle possibilité de réglage dans le plan frontal facilite la fixation de la tige de liaison **15** sur chaque ensemble de fixation **2.** La connexion de la tige de liaison **15** est encore facilitée par la possibilité de réglage, dans le plan transversal coupant l'axe du rachis, de la position du collier de réception **23.** Le système de fixation **14** pour la tige de liaison **15,** comporte ainsi une possibilité de réglage tridimensionnel permettant de faciliter la connexion de la tige de liaison **15** tout en réduisant les contraintes mécaniques imposées aux éléments d'ancrage osseux **3.** A cet égard, il est à noter que la fixation de la tige de liaison **15** sur chaque barre de connexion **6,** conduit à répartir les efforts. Cette répartition des efforts est avantageusement obtenue pour chaque ensemble **2** comportant deux éléments d'ancrage osseux **3** sur lesquels se répartissent les efforts. Les contraintes mécaniques imposées à chaque élément d'ancrage osseux **3** sont donc réduites par rapport au système antérieur dans lequel chaque élément d'ancrage osseux subit les contraintes imposées par une tige de liaison. Les contraintes mécaniques, appliquées sur les éléments d'ancrage osseux **3,** sont limitées à celles générées essentiellement lors de l'action correctrice ou stabilisatrice exécutée.

Par ailleurs, il est à noter que le dispositif de liaison intervertébrale **1** nécessite l'utilisation d'une unique tige de liaison **15.** Bien entendu, il peut être envisagé de monter sur une barre de connexion **6** d'un ensemble de fixation **2,** les extrémités de deux tiges de liaison **15** s'étendant en sens inverse l'une de l'autre. Un tel dispositif de liaison 1 peut ainsi être installé sur des segments du rachis séparés ou contigus.

Il est à noter que chaque ensemble de fixation **2** conforme à l'invention peut être associé à d'autres éléments d'ancrage osseux, tels que des crochets. Dans le même sens, il est à noter que les éléments d'ancrage osseux **3** peuvent être constitués de manière différente comme par exemple, par l'intermédiaire d'un crochet vertébral simple ou double. Ainsi, tel que cela ressort de la **fig. 1,** au moins un et dans l'exemple illustré, deux éléments d'ancrage osseux **3** sont constitués chacun par un crochet double, tel que celui décrit dans le brevet FR 2 763 236.

Dans la description qui précède, le système de fixation **14** est constitué essentiellement à partir de deux colliers **19, 23** sensiblement identiques formant une sorte de croisillon. Bien entendu, le système de fixation **14** peut être réalisé de manière différente.

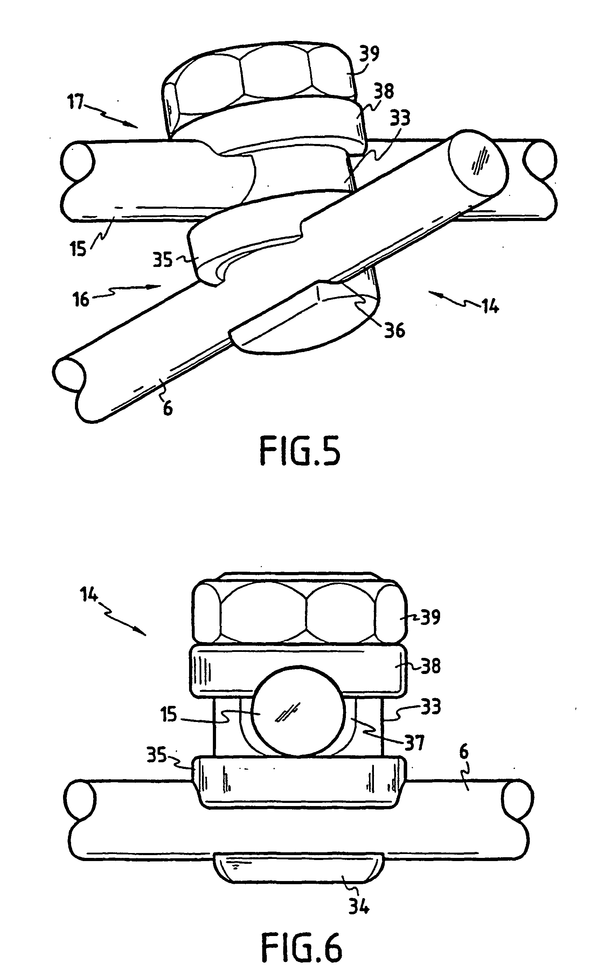

Les **fig. 5** et **6** décrivent, à titre d'exemple, une deuxième variante de réalisation du système de fixation **14** comportant des moyens de montage **16** constitués par un bloc-support **33,** de forme générale cylindrique, présentant, à son extrémité inférieure, une mâchoire **34** délimitant avec une bague d'appui **35** un logement de passage **36** pour la barre de connexion **6.** La bague d'appui **35** est montée en coulissement libre sur le bloc-support **33.** Le bloc-support **33** comporte un logement de passage **37** pour la tige de liaison **15.** De préférence, le logement de passage **37** présente une section droite de passage supérieure à la section droite transversale de la tige de liaison **15** pour autoriser un réglage en débattement angulaire. Les moyens de fixation **17** pour la tige de liaison **15** sont constitués par une rondelle coiffante **38** montée sur l'extrémité supérieure du bloc-support **33,** de sorte que la tige de liaison **15** se trouve enserrée entre la rondelle coiffante **38** et la bague d'appui **35.** L'extrémité du bloc-support **33** est filetée pour recevoir un écrou **39** agissant sur la rondelle coiffante **38** pour assurer par serrage, l'immobilisation de la tige de liaison **15** sur le système de fixation **14** et dudit système **14** sur la barre de connexion **6.**

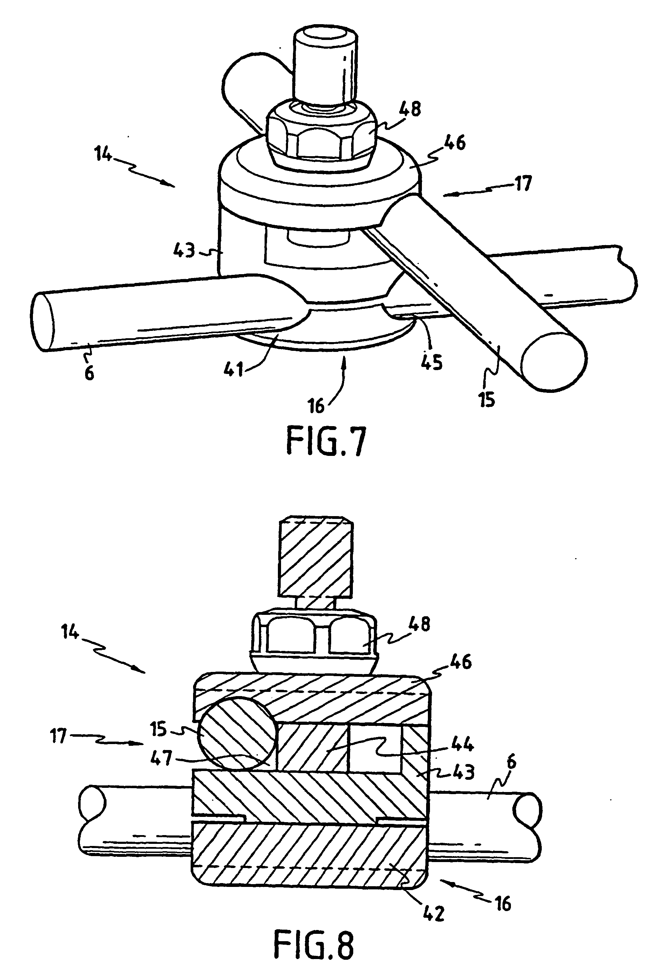

Les **fig. 7** et **8** décrivent une troisième variante de réalisation d'un système de fixation **14** comportant des moyens de montage **16** constitués par un étrier **41** formé entre un corps **42** et une coupelle intermédiaire **43.** Le corps **42** se présente sous la forme d'un disque surmonté d'un prolongement **44** sur lequel est montée, en position de superposition, la coupelle intermédiaire **43.** Le disque **42** et la coupelle intermédiaire **43** sont conformés pour délimiter entre eux, un logement de passage **45** pour la barre de connexion **6.**

Les moyens de fixation **17** sont constitués par une coupelle d'appui **46** montée sur le prolongement **44** du corps et venant coiffer la coupelle intermédiaire **43** en délimitant entre elles, un logement de passage **47** pour la tige de liaison **15.** Tel que cela ressort des dessins, le logement de passage **47** se trouve décalé par rapport à l'axe du prolongement **44.** La coupelle d'appui **46** est surmontée par un écrou **48** vissé sur un filetage réalisé sur le prolongement **44** du corps. Le serrage de l'écrou **48** conduit au blocage du système de fixation **14** sur la barre de connexion **6** et de la tige de liaison **15** sur le système de fixation **14.**

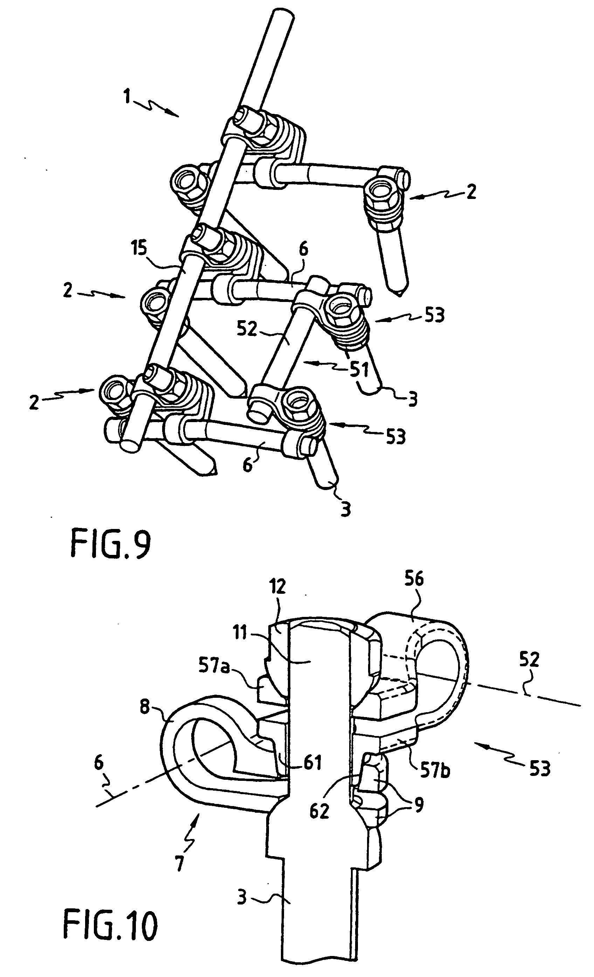

Les fig. **9** et **10** illustrent une variante du dispositif **1** de liaison intervertébrale conformes à l'invention permettant d'augmenter la tenue mécanique entre au moins deux ensembles de fixation **2** à l'aide d'au moins un système d'entretoisement **51.** Dans l'exemple illustré, le dispositif **1** comporte un système d'entretoisement **51** entre deux ensembles de fixation **2** voisins. Le système d'entretoisement **51** assure une liaison entre les barres de connexion **6** des ensembles de fixation **2.** Le système d'entretoisement **51** comporte une tige de liaison complémentaire **52,** équipée de moyens **53** de montage en position fixe sur deux éléments d'ancrage **3** situés en vis-à-vis et appartenant aux deux ensembles de fixation **2.**

De manière préférée, les moyens de montage **53** possèdent un possibilité de rotation autour d'un axe de pivotement confondu avec l'axe de l'élément d'ancrage **3.** Tel que cela apparaît sur la fig. **10,** les moyens de montage **53** de la tige de liaison complémentaire **52** sur chaque élément d'ancrage **3** sont constitués par un collier ouvert **56** de passage pour la tige de liaison complémentaire **52.** Chaque collier ouvert **56** est prolongé par deux branches, respectivement supérieure 57a et inférieure **57b** conformées chacune sous la forme d'une bague destinée à être insérée sur le prolongement fileté **11** de l'élément d'ancrage **3,** par dessus les branches **9** du collier **8** de réception de la barre de connexion **6.** De préférence, les moyens de montage **53** et les moyens de guidage et blocage **7** sont aménagés pour comporter des moyens assurant un calage angulaire entre eux. Ces moyens sont du type à emboîtement conique et sont formés par un cône **61** s'étendant à partir de la face inférieure de la branche inférieure **57b** du collier **56** et coopère avec un alésage **62** tronconique délimitant l'intérieur de la bague supérieure **9** du collier **8.** Le serrage de l'écrou **12** sur l'extrémité du prolongement fileté **11** conduit à l'enfoncement mutuel, entre le cône **61** et l'alésage tronconique **62,** assurant un blocage angulaire entre les deux colliers **8, 56** et un blocage de la barre de connexion **6** et de la tige de liaison complémentaire **52,** respectivement, par les colliers **8, 56.**

Un tel système d'entretoisement **51** permet d'assurer une liaison entre deux barres de connexion **6,** avec une possibilité de réglage de l'orientation de la tige de liaison complémentaire **52** par rapport à la direction passant par les deux vis d'ancrage supportant les colliers de montage **56.** De préférence, le système d'entretoisement **51** est monté sur les vis d'ancrage **3** qui sont les plus éloignées de la tige de liaison **15.** La tige de liaison **15** et la tige de liaison complémentaire **52** constituent ainsi, avec les barres de connexion **6** une sorte de cadre de rigidification permettant de maintenir, en position fixe déterminée, la structure ainsi constituée.

Dans l'exemple illustré ci-dessus, la tige de liaison complémentaire **52** est montée sur deux ensembles de fixation **2.** Bien entendu, la tige de liaison complémentaire **52** peut être montée sur un nombre plus important d'ensembles de fixation **2.** Dans le même sens, il peut être envisagé de monter la tige de liaison complémentaire **52** sur des ensembles de fixation qui ne sont pas voisins.

## Revendications

1. Dispositif de liaison intervertébrale, comportant au moins un ensemble de fixation **(2)** comprenant :
- au moins un élément d'ancrage osseux **(3)** destinés à être monté sur une même vertèbre,
- une barre de connexion **(6)** présentant au moins une courbure et montée sur chaque élément d'ancrage **(3)** par l'intermédiaire de moyens **(7)** de guidage en rotation de ladite barre autour de son axe et de blocage dans une position fixe déterminée,
- et un système (**14**) de fixation pour au moins une tige de liaison **(15)**, comportant des moyens **(16)** pour son montage dans une position déterminée sur la barre de connexion **(6),** ces moyens de montage **(16)** étant équipés de moyens de fixation **(17)** pour la tige de liaison **(15),** de manière à assurer la solidarisation de ladite tige de liaison dans une position déterminée par rapport à l'élément d'ancrage **(3)** en vue d'assurer une correction vertébrale,
**caractérisé en ce que** les moyens de montage **(16)** du système de fixation **(14)** sur la barre de connexion, et les moyens de fixation **(17)** pour la tige de liaison sont constitués par l'intermédiaire d'un premier **(19)** et deuxième **(23)** colliers ouverts de passage, respectivement de la barre de connexion **(6)** et de la tige de liaison **(15),** chaque collier ouvert **(19, 23)** étant muni de moyens de blocage **(18)** permettant d'assurer l'immobilisation du système de fixation **(14)** sur la barre de connexion **(6)** et de la tige de liaison **(15)** par rapport au système de fixation.

2. Dispositif selon la revendication 1, **caractérisé en ce qu'**il comporte au moins un ensemble de fixation **(2)** comprenant une paire d'éléments d'ancrage osseux **(3)** destinés à être montés sur une même vertèbre ou sur deux vertèbres adjacentes, la barre de connexion **(6)** étant fixée entre les éléments d'ancrage **(3)** d'une même paire.

3. Dispositif selon la revendication 1, **caractérisé en ce que** les moyens de fixation **(17)** pour la tige de liaison **(15)** sont montés sur les moyens de montage **(16)** avec une possibilité de rotation autour d'un axe de pivotement.

4. Dispositif selon la revendication 3, **caractérisé en ce que** les moyens de montage **(16)** et les moyens de fixation **(17)** sont aménagés pour comporter des moyens de calage angulaire **(26)** assurant un blocage entre eux.

5. Dispositif selon la revendication 1, **caractérisé en ce que** les moyens de montage (**16**) du système de fixation (**14**) sur la barre de connexion (**6**) assurent un coulissement et un pivotement du système de fixation **(14)** autour de l'axe de la barre de connexion **(6).**

6. Dispositif selon la revendication 1, **caractérisé en ce que** les moyens de blocage **(18)** pour les colliers de réception **(19, 23)** constituent une seule pièce.

7. Dispositif selon la revendication 6, **caractérisé en ce que** les moyens de blocage **(18)** pour les colliers de réception **(19, 23)** sont constitués par un écrou coopérant avec une tige filetée **(25)** s'étendant à partir d'une branche **(21a)** du premier **(19)** collier, tandis que l'autre branche **(21b)** dudit collier est traversée par la tige filetée **(25)** sur laquelle sont emmanchées des bagues **(24a, 24b)** s'étendant à partir du deuxième collier **(23)** et destinées à être coiffées par l'écrou **(18)** permettant d'assurer le blocage de la barre de connexion et de la tige de liaison.

8. Dispositif selon la revendication 1, **caractérisé en ce que** les moyens (**7**) de guidage et de blocage en rotation de la barre de connexion (**6**) sont constitués par un collier ouvert **(8)** de passage pour la barre de connexion **(6),** prolongé par deux branches **(9)** conformées chacune sous la forme d'une bague destinée à être insérée sur un prolongement fileté **(11)** faisant partie de la tête de fixation **(5)** d'un élément d'ancrage (**3**) et destinée à recevoir un écrou de blocage (**12**).

9. Dispositif selon la revendication 1, **caractérisé en ce qu'**il comporte en tant qu'élément d'ancrage osseux (**3**), une vis pédiculaire ou un crochet simple ou double.

10. Dispositif selon l'une des revendications 1 à 9, **caractérisé en ce qu'**il comporte une série d'ensembles de fixation (**2**) montés chacun sur au moins une vertèbre donnée et reliés entre eux par l'intermédiaire d'au moins une tige de liaison (**15**).

11. Dispositif selon la revendication 10, **caractérisé en ce qu'**il comporte, pour au moins deux ensembles de fixation (**2**) montés sur deux vertèbres, au moins un système d'entretoisement entre les barres de connexion **(6)** appartenant auxdits ensembles de fixation, chaque système d'entretoisement comportant une tige de liaison complémentaire pourvue de moyens de montage en position fixe sur les éléments d'ancrage **(3)** situés en vis à vis et appartenant aux ensembles de fixation **(2).**

12. Dispositif selon la revendication 11, **caractérisé en ce que** les moyens de montage en position fixe de la tige de liaison complémentaire sur les éléments d'ancrage **(3)** possèdent une possibilité de rotation autour d'un axe de pivotement confondu avec l'axe de l'élément d'ancrage (**3**).

13. Dispositif selon la revendication 12, **caractérisé en ce que** les moyens de montage en position fixe de la tige de liaison complémentaire sur chaque élément d'ancrage **(3)** sont constitués par un collier ouvert de passage pour la tige de liaison complémentaire, prolongé par deux branches conformées chacune sous la forme d'une bague destinée à être insérée sur un prolongement fileté faisant partie de la tête de fixation **(5)** d'un élément d'ancrage et passant également dans les branches du collier de passage pour la barre de connexion **(6),** le prolongement fileté étant destiné à recevoir un écrou de blocage.

## Patentansprüche

1. Zwischenwirbelverbindungseinrichtung, welche wenigstens eine Fixierungsanordnung (2) aufweist, umfassend:
- wenigstens ein Knochonverankerungselement (3), das dazu bestimmt ist, auf ein und demselben Wirbel befestigt zu werden,
- eine Verbindungsstange (6), die wenigstens eine Krümmung aufweist und auf jedem Verankerungselement (3) mit Hilfe von Mitteln (7) zum Führen bei der Drehung der Stange um ihre Achse und zum Blockieren in einer bestimmten festen Position befestigt ist,
- und ein System (14) zum Befestigen für wenigstens einen Verbindungsstab (15), der Mittel (16) aufweist, um ihn in einer bestimmten Position auf der Verbindungsstange (6) zu befestigen, wobei die Befestigungsmittel (16) mit Fixierungsmitteln (17) für den Verbindungsstab (15) derart versehen sind, daß die formschlüssige Verbindung des Verbindungsstabes in einer bestimmten Position in Bezug auf das Verankerungselement (3) gewährleistet wird, um eine Wirbelkorrektur sicherzustellen,
**dadurch gekennzeichnet, daß** die Befestigungsmittel (16) des Fixierungssystems (14) auf der Verbindungsstange und die Fixierungsmittel (17) für den Verbindungsstab mittels einer ersten (19) und einer zweiten (23) offenen Durchgangsschelle der Verbindungsstange (6) bzw. des Verbindungsstabs (15) gebildet sind, wobei jede offene Schelle (19, 23) mit Blocklerungsmitteln (18) versehen ist, welche das Feststellen des Fixierungssystems (14) auf der Verbindungsstange (8) und des Verbindungsstabs (15) in Bezug auf das Fixierungssystem gewährleisten.

2. Einrichtung nach Anspruch 1, **dadurch gekennzeichnet, daß** sie wenigstens eine Fixierungsanordnung (2) aufweist, die ein Paar Knochenverankerungselemente (3) umfaßt, die dazu bestimmt sind, auf ein und demselben Wirbel oder auf zwei benachbarten Wirbeln befestigt zu werden, wobei die Verbindungsstange (6) zwischen den Verankerungselementen (3) eines selben Paares fixiert ist.

3. Einrichtung nach Anspruch 1, **dadurch gekennzeichnet, daß** die Fixierungsmittel (17) für den Verbindungsstab (15) auf den Befestigungsmitteln (16) befestigt sind, wobei die Möglichkeit zum Drehen um eine Drehachse besteht.

4. Einrichtung nach Anspruch 3, **dadurch gekennzeichnet, daß** die Befestigungsmittel (16) und die Fixierungsmittel (17) so angeordnet sind, daß sie Mittel (26) zum winkeligen Verkeilen aufweisen, die ein Blockieren zwischen ihnen gewährleisten.

5. Einrichtung nach Anspruch 1, **dadurch gekennzeichnet, daß** die Befestigungsmittel (16) des Fixierungssystems (14) auf der Verbindungsstange (6) ein Gleiten und ein Drehen des Fixierungssystems (14) um die Achse der Verbindungsstange (6) herum gewährleisten.

6. Einrichtung nach Anspruch 1, **dadurch gekennzeichnet, daß** die Blockierungsmittel (18) für die Aufnahmeschellen (19, 23) ein einziges Stück bilden.

7. Einrichtung nach Anspruch 6, **dadurch gekennzeichnet, daß** die Blockierungsmittel (18) für die Aufnahmeschellen (19, 23) aus einer Mutter bestehen, die mit einem Gewindestift (25) zusammenwirkt, der sich ausgehend von einem Zweig (21a) der ersten Schelle (19) erstreckt, während der andere Zweig (21b) der Schelle von dem Gewindestift (25) durchquert wird, auf dem Ringe (24a, 24b) aufgezogen sind, die sich ausgehend von der zweiten Schelle (23) erstrecken und dazu bestimmt sind, durch die Mutter (18) bedeckt zu werden, wodurch das Blockieren der Verbindungsstange und des Verbindungsstabs gewährleistet wird,

8. Einrichtung nach Anspruch 1, **dadurch gekennzeichnet, daß** die Mittel (7) zum Führen und Blockieren beim Drehen der Verbindungsstange (6) aus einer offenen Durchgangsschelle (8) für die Verbindungsstange (6) gebildet sind, die um zwei Zweige (9) verlängert ist, welche Jeweils in Form eines Ringes ausgebildet sind, der dazu bestimmt ist, auf eine Gewindeverlängerung (11) aufgezogen zu werden, die Teil des Fixierungskopfes (5) eines Verankerungselementes (3) ist, und dazu, eine Blockierungsmutter (12) aufzunehmen.

9. Einrichtung nach Anspruch 1, **dadurch gekennzeichnet, daß** sie als Knochenverankerungselement (3) eine pedikuläre Schraube oder einen Einfach- oder Doppelhaken aufweist.

10. Einrichtung nach Anspruch 1 bis 9, **dadurch gekennzeichnet, daß** sie eine Reihe von Fixierungsanordnungen (2) aufweist, die jeweils auf wenigstens einem bestimmten Wirbel befestigt und untereinander mit Hilfe von wenigstens einem Verbindungsstab (15) verbunden sind.

11. Einrichtung nach Anspruch 10, **dadurch gekennzeichnet, daß** sie für wenigstens zwei Befestigungsanordnungen (2), die auf zwei Wirbeln befestigt sind, wenigstens ein Versteifungssystem zwischen den Verbindungsstangen (6) aufweist, die zu den Fixierungsanordnungen gehören, wobei jedes Versteifungssystem einen zusätzlichen Verbindungsstab aufweist, der mit Mitteln zum Befestigen in fester Position auf den Verankerungselementen (3) versehen ist, die gegenüberliegend angeordnet sind und zu den Fixierungsanordnungen (2) gehören.

12. Einrichtung nach Anspruch 1, **dadurch gekennzeichnet, daß** die Mittel zum Befestigen des zusätzlichen Verbindungsstabs in fester Position auf den Verankerungselementen (3) eine Drehung um eine Drehachse herum ermöglichen, die in die Achse des Verankerungselementes (3) übergeht.

13. Einrichtung nach Anspruch 12, **dadurch gekennzeichnet, daß** die Mittel zum Befestigen des zusätzlichen Verbindungsstabs in fester Position auf jedem Verankerungselement (3) aus einer offenen Durchgangsschelle für den zusätzlichen Verbindungsstab bestehen, die durch zwei Zweige verlängert ist, die jeweils in Form eines Ringes ausgebildet sind, der dazu bestimmt ist, auf eine Gewindeverlängerung aufgezogen zu werden, die Teil des Fixierungskopfes (5) eines Verankerungselementes ist und auch in den Zweigen der Durchgangsschelle für die Verbindungsstange (6) verläuft, wobei die Gewindeverlängerung dazu bestimmt ist, eine Blockierungsmutter aufzunehmen.

## Claims

1. An intervertebral linking device including at least one attachment assembly (2) comprising:
- at least one bone anchoring component (3) intended to be mounted on a same vertebra,
- a connecting bar (6) having at least one curvature and mounted on each anchoring component (3) via means (7) for guiding said bar in rotation around its axis and for blocking it in a determined fixed position,
- and an attachment system (14) for at least one linking rod (15), including means (16) for its mounting in a determined position on the linking rod (6), these mounting means (16) being fitted out with attachment means (17) for the linking rod (15) in order to provide permanent attachment of said linking rod in a determined position relatively to the anchoring component (3) for providing vertebral correction,
**characterized in that** the means (16) for mounting the attachment system (14) on the connecting bar and the attachment means (17) for the linking rod, via first (19) and second (23) open passage collars, consist of the connecting bar (6) and the linking rod (15), respectively, each open collar (19, 23) being provided with blocking means (18) for providing immobilization of the attachment system (14) on the connecting bar (6) and the linking rod (15) relatively to the attachment system.

2. The device according to claim 1, **characterized in that** it includes at least one attachment assembly (2) comprising a pair of bone anchoring components (3) intended to be mounted on a same vertebra or on two adjacent vertebrae, the connecting rod (6) being attached between the anchoring component (3) of a same pair.

3. The device according to claim 1, **characterized in that** the means (17) for attaching the linking rod (15) are mounted on the mounting means (16) with possibility of rotation around a pivot axis.

4. The device according to claim 3, **characterized in that** the mounting means (16) and the attachment means (17) are arranged so as to include angular blocking means (26) providing blocking of each other.

5. The device according to claim 1, **characterized in that** the means (16) for mounting the attachment system (14) on the connecting rod (6) provide sliding and pivoting of the attachment system (14) around the axis of the connecting bar (6) .

6. The device according to claim 1, **characterized in that** the blocking means (18) for the receiving collars (19, 23) consist of a single part.

7. The device according to claim 6, **characterized in that** the blocking means (18) for the receiving collars (19, 23) consist of a nut cooperating with a threaded rod (25) extending from a branch (21a) of the first (19) collar, while the other branch (21b) of said collar is crossed by the threaded rod (25) on which rings (24a, 24b) are fitted extending from the second collar (23) and intended to be capped by the nut (18) with which blocking of the connecting bar and of the linking rod may be provided.

8. The device according to claim 1 **characterized in that** the means (7) for guiding and blocking in rotation the connecting bar (6) consist of an open collar (8) for letting through the connecting bar (6), extended by two branches (9) each conformed as a ring intended to be inserted on a threaded extension (11) being part of the attachment head (5) of an anchoring component (3) and intended to receive a locking nut (12).

9. The device according to claim 1, **characterized in that** it includes a pedicular screw or a simple or double hook as a bone anchoring component (3).

10. The device according to any of claims 1 to 9, **characterized in that** it includes a series of attachment assemblies (2) each mounted on at least one given vertebra and connected with each other via at least one linking rod (15).

11. The device according to claim 10, **characterized in that** it includes, for at least two attachment assemblies (2) mounted on two vertebrae, at least one spacer system between the connecting bars (6) belonging to said attachment assemblies, each spacer system including a complementary linking rod provided with means for mounting it in a fixed position on the anchoring components (3) located facing each other and belonging to the attachment assemblies (2).

12. The device according to claim 11, **characterized in that** the means for mounting the complementary linking rod in a fixed position on the anchoring component (3) have a possibility of rotation around a pivot axis coinciding with the axis of the anchoring component (3).

13. The device according to claim 12, **characterized in that** the means for mounting the complementary linking rod in a fixed position on each anchoring component (3) consist of an open collar for letting through the complementary linking rod, extended by two conformed branches each as a ring intended to be inserted on a threaded extension being part of the attachment head (5) of an anchoring component and also passing in the branches of the passage collar for the connecting bar (6), the threaded extension being intended for receiving a locking nut.
